# EUROPEAN PATENT APPLICATION

(11) **EP 4 215 160 A1**
(43) Date of publication of application: **26.07.2023**
(21) Application number: 21868090.8
(22) Date of filing: 16.04.2021
(51) Int. Cl.: A61F 2/24

(54) **IMPLANT**

(30) Priority: 15.09.2020 CN 202010969135; 15.09.2020 CN 202010967319
(71) Applicant: Shenzhen Lifevalve Medical Scientific Co., Ltd., Shenzhen, Guangdong 518000 (CN)
(72) Inventor: ZHENG, Weicheng, Shenzhen, Guangdong 518000 (CN); LAI, Liushan, Shenzhen, Guangdong 518000 (CN); CHEN, Wenjun, Shenzhen, Guangdong 518000 (CN)
(74) Representative: Prinz & Partner mbB
(86) International application number: PCT/CN2021/087635
(87) International publication number: WO 2022/057252

(57) **Abstract**

An implant is provided, including a tubular stent structure and a skirt; the stent structure is provided with barb structures; the stent structure is radially compressible or expandable; the stent structure includes mesh structures circumferentially distributed; the skirt is arranged on a surface of the stent structure; the mesh structures include first mesh units and second mesh units; an area of the first mesh units is larger than an area of the second mesh units; a size of each second mesh unit is substantially the same as a size of each barb structure; and the barb structures can be accommodated in the second mesh units. The implant can avoid the barb structures and the skirt from being entangled and interfered with each other when the stent structure compresses and expands in loading and releasing processes.

## Description

### Field

The embodiments relate to the field of medical devices, and to an implant.

### Background

The heart is a very important organ of humans. It provides power for the blood circulation of human. A heart is divided into a left part and a right part. Each part contains a ventricle and an atrium. The ventricles are separated by a ventricular septum and the atriums are separated by an atrial septum. There are valves between the atriums, the ventricles and the arteries to prevent blood reflux. The valve between the left atrium and the left ventricle is a mitral valve; the valve between the right atrium and the right ventricle is a tricuspid valve; the valve between the left ventricle and the great artery is an aortic valve; and the valve between the right ventricle and the pulmonary artery is a pulmonary valve.

The above valves will be opened and closed with the contraction and relaxation of the heart, so the heart valves need to be able to withstand pressures from blood and surrounding valve rings and blood's flushing for a long time. If the valves cannot be fully closed or opened due to diseases or other reasons, blood reflux and insufficient blood supply will be caused. For example, valvular stenosis will lead to poor blood circulation or incomplete closure, which will lead to insufficient blood supply to the heart, thus greatly increasing a pressure on the heart and leading to a heart failure. For such heart valve diseases, in a traditional therapy, the heart stops beating after the chest is opened. Under extracorporeal circulation, the heart is opened for surgical repair of valves or replacement of artificial valves. After the surgery is completed, the heart is made to beat again, and subsequent operations are then completed. Surgical valve replacement is traumatic, and patients need long recovery time, so it is often contraindicated for elderly patients due to their ages, weak constitutions and severe lesions, or because they have other diseases.

A minimal trauma surgery technology is constantly being developed. An artificial heart valve can be introduced into the body of a patient through a catheter, that is, the heart valve can be placed through minimally invasive interventional surgery, in which, it is not necessary to open the chest, so this surgery features with small trauma and quick postoperative recovery. A new solution is provided for those patients suffering from the heart valve stenosis, who cannot count on current therapies to prolong their life or relieve their pains. However, due to its relatively short history, this technology still has many problems. For example, existing stents for interventional artificial heart valves are still in the initial stage of research. There are a small number of kinds of stents having the following defects: the stents cannot be quickly and accurately released, the stents easily move under blood pressure and blood's flushing, and the like, so that an ideal interventional treatment effect cannot be achieved. In order to solve the problem of positioning of a stent, a method of adding barbs can be adopted. After a valve is released, a barb can penetrate into a vascular wall after the heart valve is implanted to a specific position, so that the valve difficultly moves in the blood vessel. However, when the stent compresses and expands in loading and releasing processes, the barb arranged on the stent is easily interfered with a skirt. For example, the stent needs to compress into a shape with a smaller diameter during loading. At this time, the skirt structure shrinks in each grid of the stent with the compression of the stent, and the barb is easily entangled with the skirt. When the stent expands during release, the barb is entangled with the skirt, making it difficult to release the stent, which easily causes a surgical failure. Since the barb is at a certain angle relative to a valve stent tube, the barb structure will easily scratch an inner wall of the sheath in the processes of loading and releasing the valve to and from a delivery sheath. When a deliverer is implanted into the body, if a filamentous substance scraped out of the sheath remains in the body, the risk of an operation will increase.

### Summary

The embodiments aim to at least avoid the impact, caused by the entanglement between a barb and a skirt during compression and expansion of an implant in loading and releasing processes, on the release of the implant. The objective is achieved by the following way.

An implant includes a tubular stent structure and a skirt; the stent structure is provided with at least one barb structure; the stent structure is radially compressible or expandable; the stent structure includes mesh structures circumferentially distributed; the skirt is arranged on a surface of the stent structure; the mesh structures include a plurality of first mesh units and a plurality of second mesh units; an area of one of the first mesh units is larger than an area of one of the second mesh units; a size of one of second mesh units is substantially the same as a size of one barb structure; and one barb structures can be accommodated in the one of second mesh units.

In one embodiment, a junction is provided between two adjacent first mesh units; and each second mesh unit is arranged at the junction.

In one embodiment, the first mesh units are of hexagonal structures; and one edge of one hexagonal structure overlaps one edge of the other hexagonal structure to form the junction.

In one embodiment, each first mesh unit is provided with at least one arc-shaped bulge structure along an axial direction of the stent structure; and the arc-shaped bulge structure protrudes towards a direction away from the first mesh unit.

In one embodiment, each barb structure includes a connection end, a first position and a free end; the connection end is connected to the junction; the free end extends out of the second mesh unit; the first position is located between the connection end and the free end; and an angle formed between a straight line formed by the connection end and the first position and a central axis of the stent structure is greater than an angle formed between a straight line formed by the connection end and the free end and the central axis of the stent structure.

In one embodiment, the barb structure includes a first section and a second section connected to each other; the first section is between the connection end and the first position; the second section is between the first position and the free end; and the free end is a sharp end structure.

In one embodiment, the first section is a straight bar-shaped structure, and a first surface of the second section facing an outer side of the stent structure is provided with an arc-shaped structure.

In one embodiment, the first section is a straight bar-shaped structure or a curved structure; and the second section is a curved structure.

In one embodiment, the barb structures and the stent structure are connected together after being formed respectively, or the barb structures and the stent structure are integrally formed by cutting a tubular product.

In one embodiment, the implant is a heart valve; the heart valve further includes a valve structure; the valve structure including a plurality of valve leaflets fitting one another; each valve leaflet includes a flange; and the flange passes through the second mesh unit in a direction from the inside of the stent structure to the outside.

According to the implant, the second mesh units capable of accommodating the barb structures are provided. The second mesh units have a small area, and the size of the second mesh units is substantially the same as that of the barb structures, so that the second mesh units just accommodate the barb structures. The skirt located in the second mesh units are hardly entangled with the barb structures because of its small area and small shrinkage degree, thus avoiding mutual interference between the barb structures and the skirt when the stent structure compresses and expands in the loading and releasing processes.

An implant includes a tubular stent structure and at least one barb structures arranged on the stent structure; the stent structure includes a plurality of mesh structures circumferentially distributed; one barb structures can be accommodated in one of the mesh structures; each barb structure includes an elastic element;
one end of each elastic element is connected to each mesh structure to form a connection end;
the elastic element is configured to outwards protrude from the stent structure to form a first position and then bend from the first position to the stent structure;
a window structure is arranged on the elastic element; a thorn-like structure connected to the window structure is arranged in the window structure; the thorn-like structure has a free end extending from the window structure to a direction away from the stent structure; and an angle formed between a straight line formed by the connection end and the first position and a central axis of the stent structure is greater than an angle formed between a straight line formed by the connection end and the free end and the central axis of the stent structure.

In one embodiment, the connection ends are connected to distal ends of the mesh structures, and the thorn-like structures are connected to proximal ends of the window structures; or the connection ends are connected to proximal ends of the mesh structures, and the thorn-like structures are connected to distal ends of the window structures.

In one embodiment, the mesh structures include a plurality of first mesh units and a plurality of second mesh units; an area of one of the first mesh units is larger than an area of one of the second mesh units; a size of one of second mesh units is substantially the same as a size of one barb structure; and one barb structure can be accommodated in the one of second mesh units.

In one embodiment, a junction is provided between two adjacent first mesh units; and each second mesh unit is arranged at the junction.

In one embodiment, the first mesh units are of hexagonal structures; and one edge of one hexagonal structure overlaps one edge of the other hexagonal structure to form the junction.

In one embodiment, the stent structure is radially compressible or expandable; as the stent structure radially compresses or expands, the first mesh units circumferentially compress or expand along the stent structure; each first mesh unit is provided with at least one arc-shaped bulge structure along an axial direction of the stent structure; and the arc-shaped bulge structure protrudes towards a direction away from the first mesh unit.

In one embodiment, the free end is a sharp end structure.

In one embodiment, at least one barb structure and the stent structure are connected into a whole after being respectively formed.

In one embodiment, at least one barb structure and the stent structure are integrally formed by cutting a tubular product.

In one embodiment, the implant is a heart valve; the heart valve further includes a valve structure; the valve structure includes a plurality of valve leaflets fitting one another; each valve leaflet includes a flange; and the flange passes through the second mesh unit in a direction from the inside of the stent structure to the outside, and is then sutured on the skirt.

According to the above implant, the barb structures can be accommodated in the mesh structures. The barb structures include elastic structures having the connection ends, the first positions and the window structures. The thorn-like structures are arranged in the window structures and have the free ends extending from the window structures in the direction away from the stent structure. The angle formed between the straight line formed by the connection end and the first position and the central axis of the stent structure is greater than the angle formed between the straight line formed by the connection end and the free end and the central axis of the stent structure. In this way, the following can be ensured: when a sheath presses the connection ends of the elastic structures to be flush with the mesh structures, the free ends of the thorn-like structures are not exposed. When the sheath releases the implant, the free ends of the thorn-like structures will be released only when the connection ends of the barb structures extend out of the mesh structures, so that the following phenomenon is avoided: In the loading, releasing or withdrawing process, the free ends of the thorn-like structures easily scratch out filamentous debris from the inner wall of the sheath, and the debris easily causes an immunity risk after the implant is implanted into the body of a user.

### Brief Description of the Drawings

By reading the detailed description in the implementation modes below, various other advantages and benefits will become clear to those of ordinary skill in the art. The accompanying drawings are only used for the purpose of illustrating the implementation modes and are not considered as a limitation to the embodiments. Furthermore, throughout the drawings, the same reference numerals are used to denote the same components. In the drawings:
Fig. 1 is a schematic diagram of a heart valve provided according to one of the embodiments.
Fig. 2 is a schematic structural diagram of a stent in Fig. 1.
Fig. 3 is a schematic structural diagram of a valve in Fig. 1.
Fig. 4 is an enlarged diagram of A in Fig. 2.
Fig. 5 is a schematic structural diagram of cooperation between a barb structure and a second mesh unit in another embodiment.
Fig. 6 is a schematic diagram of another view of Fig. 5.
Fig. 7 is a schematic structural diagram of cooperation between a barb structure and a second mesh unit in still another embodiment.
Fig. 8 is a schematic diagram of another view of Fig. 7.

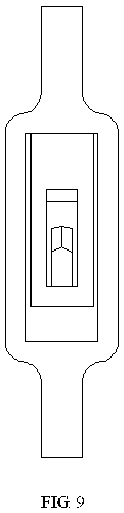

Fig. 9 is a schematic diagram of still another view of Fig. 7.

### Detailed Description of the Embodiments

Exemplary implementations of the embodiments will be described in more detail below with reference to the accompanying drawings. Although the exemplary implementations of the embodiments are shown in the drawings, it should be understood that the embodiments can be implemented in various forms and should not be limited by the implementations set forth herein. On the contrary, these implementations are provided to enable a more thorough understanding of the embodiments and to fully deliver the scope of the embodiments to those skilled in the art.

It should be understood that the terms used herein are only for the purpose of describing specific example implementation modes, and are not intended to be limitations. Unless the context clearly indicates otherwise, the singular forms "a", "an" and "the" as used in the text may also mean that the plural forms are included. The terms "comprise", "include", "contain" and "has" are inclusive and therefore indicate the existence of the stated features, steps, operations, elements and/or components, but do not exclude the existence or addition of one or more of other features, steps, operations, elements, components, and/or combinations thereof. The method steps, processes, and operations described herein are not interpreted as requiring them to be executed in a specific order described or illustrated, unless the order of execution is clearly indicated. It should also be understood that additional or alternative steps may be used.

Although the terms first, second, third, etc. may be used in the text to describe multiple elements, components, regions, layers and/or sections, these elements, components, regions, layers and/or sections should not be restricted by these terms. These terms may only be used to distinguish one element, component, region, layer or section from another region, layer or section. Unless the context clearly indicates otherwise, terms such as "first", "second" and other numerical terms do not imply an order or a sequence when used in the text. Thus, a first element, component, region, layer or section discussed below could be referred to as a second element, component, region, layer or section without departing from the teachings of example implementations.

For ease of description, spatially relative terms may be used herein to describe a relationship of one element or feature to another element or feature as shown in the figures, such as "inner", "outer", "inside", "outside", "below", "under", "over", "above", etc. This spatially relative term is intended to include different orientations of a device in use or operation other than the orientation depicted in the figures. For example, if the device in the figures flips over, elements described as being "below or under other elements or features" would then be oriented as being "over or "above other elements or features". Thus, the example term "below" can include both upper and lower orientations. The device may be otherwise oriented (rotated 90 degrees or in other orientations) and is correspondingly interpreted with the spatially relative descriptors used herein.

It should be noted that "distal end" and "proximal end" location terms, which are commonly used in the field of interventional medical instruments. "Distal end" refers to an end far away from an operator during surgery, and "proximal end" refers to an end close to the operator during surgery. In the embodiments of the embodiments, an implant may be a heart valve, a covered stent, an occluder, and the like. After the heart valve is implanted into the body of a user, the blood enters the heart valve from a blood inflow end and flows out from a blood outflow end through the interior of the heart valve interior.

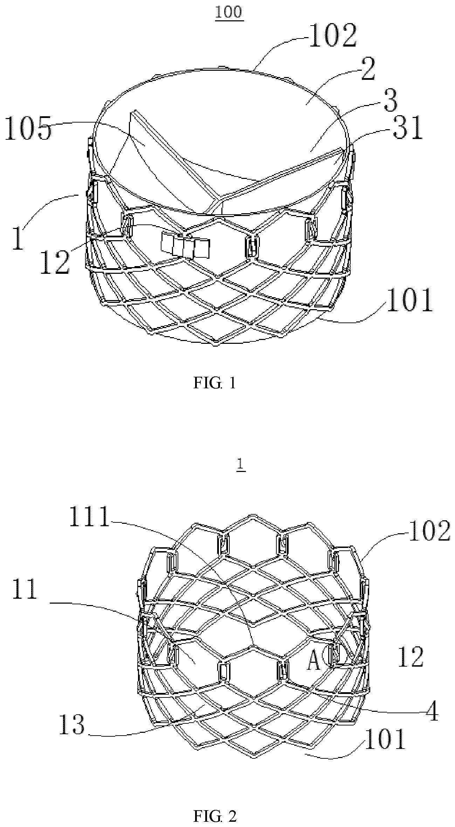

Referring to Fig. 1, one embodiment provides a heart valve 100. The heart valve 100 includes a stent structure 1, a skirt 2 and a valve structure 3. The stent structure 1 is of a tubular structure. The stent structure 1 includes an inflow end 101 and an outflow end 102. The skirt 2 is made of planar or cylindrical polyester cloth. The skirt 2 is sutured on an inner or outer surface of the expanded stent structure 1. The valve structure 3 is arranged on the inner surface of the stent structure and sutured on the skirt 2. The valve structure 3 includes at least two valve leaflets 31.

In Fig. 2, the stent structure 1 includes mesh structures circumferentially distributed and including first mesh units 11 and second mesh units 12. An area of the first mesh units 11 is larger than that of the second mesh units 12. A junction is provided between two adjacent first mesh units 11. Each second mesh unit 12 is arranged at the junction. Barb structures 4 are arranged in the second mesh units 12. A size of each second mesh unit 12 is substantially the same as that of each barb structure 4. The barb structures 4 can be accommodated in the second mesh units 12.

Being substantially the same in this embodiment means that there is a certain space between the barb structure 4 and the second mesh unit 12 after the barb structure 4 is accommodated to the second mesh unit 12.

In this embodiment, the first mesh units 11 and the second mesh units 12 are circumferentially distributed at the outflow end 102 of the stent structure 1. In other embodiments, the first mesh units 11 and the second mesh units 12 can be randomly distributed at any position of the stent structure 1. Each first mesh unit 11 is a hexagonal structure. One edge of one hexagonal structure overlaps one edge of another hexagonal structure to form a junction. Each second mesh unit 12 is arranged at the junction. Each second mesh unit 12 is of a rectangular structure. In other embodiments, the shapes of the first mesh unit 11 and the second mesh unit 12 are not limited. For example, the first mesh unit 11 can also be octagonal, and the second mesh unit 12 can also be elliptical, square, triangular, and the like, as long as the barb structure 4 can be accommodated in the second mesh unit 12. In other embodiments, two adjacent first mesh units 11 are connected through a connector. The two adjacent first mesh units 11 are independent of each other and do not overlap. The second mesh units 12 are arranged on the connectors.

If there are no second mesh units 12, the barb structures 4 are directly arranged on the first mesh units 11. The first mesh units 11 provide a suitable radial force for the stent structure 1. The area of the first mesh units 11 cannot be too small, otherwise, the radial force of the stent structure 1 will be too high, and the stent structure will not easily compress into a sheath. However, if the first mesh units 11 have a large area, the skirt portion located in the first mesh units 11 will shrink together when the stent structure 1 compresses, so the skirt portion is easily entangled with the barb structures 4 arranged on the first mesh units 11. The stent structure 1 will expand before being released, but it is difficult to release the stent structure 1 as the barb structures 4 and skirt 2 are entangled, easily leading to an operation failure. The second mesh units 12 capable of accommodating the barb structures 4 are provided. The second mesh units 12 have a small area, and the size of the second mesh units 12 is substantially the same as that of the barb structures 4, so that the second mesh units just accommodate the barb structures 4. The skirt located in the second mesh units 12 are hardly entangled with the barb structures 4 because of its small area and small shrinkage degree, thus avoiding mutual interference between the barb structures 4 and the skirt 2 when the stent structure 1 compresses and expands in the loading and releasing processes.

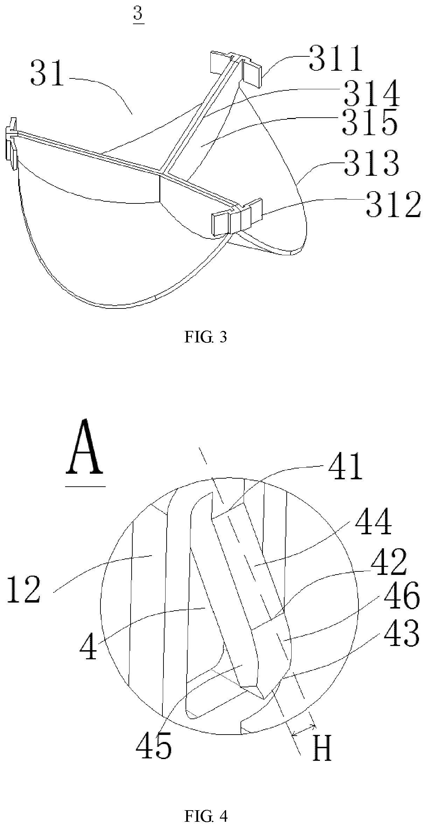

As shown in Fig. 3, the valve structure 3 includes three valve leaflets 31. Each valve leaflet 31 includes a flange 311/312, a suture edge 313 and an opening edge 314. The opening edges 314 of the three valve leaflets 31 fit each other to form a fitting surface 315. In combination with Fig. 1 and Fig. 3, the suture edges 313 are sutured on an inner wall of skirt 2. The flanges 311/312 pass through the second mesh units 12 in a direction from the inside of the stent structure 1 to the outside, and then are sutured on an outer wall of the skirt 2 along a circumferential direction. The fitting surface 315 of the opening edges 314 faces the outflow end 102, so that the blood can only flow in from the inflow end 101 and flow out from the outflow end 102 without reflux. It should be noted that in this embodiment, the second mesh units 12 can be used not only for arranging the barb structures 4, but also for fixing the flanges 311/312.

Each first mesh unit 11 is provided with at least one arc-shaped bulge structure 111 along an axial direction of the stent structure 1. The arc-shaped bulge structure 111 protrudes towards a direction away from the first mesh unit 11. When the stent structure 1 radially compresses, the first mesh element 11 is not completely closed due to the existence of the arc-shaped bulge structure 111, but there is a gap. The gap can provide a larger accommodation space for the valve leaflet 31 near the arc-shaped bulge structure 111, thus reducing folds of the valve leaflet 31 when the stent structure 1 compresses.

In this embodiment, the mesh structures also include third mesh structures 13. Each third mesh structure 13 may be polygonal, such as rhomboid or hexagonal. The third mesh structures 13 are spliced with the first mesh units 11 and the second mesh units 12 and are distributed along the circumferential direction of the stent structure 1. The splicing form is not limited.

Referring to Fig. 4, each barb structure 4 includes a connection end 41, a first position 42 and a free end 43. The connection end 41 is connected to the second mesh unit 12. The free end 43 can extend out of the second mesh unit 12. The first position 42 is located between the connection end 41 and the free end 43. An angle formed between a straight line formed by the connection end 41 and the first position 42 and a central axis of the stent structure 1 is greater than an angle formed between a straight line formed by the connection end 41 and the free end 43 and the central axis of the stent structure 1. It should be noted that the angle formed between the straight line formed by the connection end 41 and the first position 42 and the central axis of the stent structure 1 is greater than the angle formed between the straight line formed by the connection end 41 and the free end 43 and the central axis of the stent structure 1, which can mean that: the straight line formed by the connection end 41 and the first position 42 is located at a higher place relative to the free end 43. This can ensure that when the sheath presses the connection end 41 of the barb structure 4 into the second mesh unit 12 to be flush with a stent lever, the free end 43 will not be exposed. When the stent structure 1 is released from the sheath, the free end 43 will be released only when the connection end 41 of the barb structure 4 is released. In this way, the following phenomenon can be avoided: in the loading, releasing or withdrawing process, the free ends of the barb structures easily scratch out filamentous debris from the inner wall of the sheath, and the debris remains in the blood vessel for body circulation after a deliverer is implanted into the body of a user, easily causing an immunity risk.

In this embodiment, the barb structure 4 also includes a first section 44 and a second section 45 which are connected with each other. The first section 44 is between the connection end 41 and the first position 42, and the second section 45 is between the first position 42 and the free end 43. The free end 43 is a sharp end structure, which is convenient for the valve to pierce the vascular wall in a positioning process. In other embodiments, the sharp end structure may not be provided because the barb structure 4 itself is small and easily punctures into a tissue. The first section 44 is a straight bar-shaped structure, and a first surface 46 of the second section 45 facing an outer side of the stent structure is an arc-shaped structure. The arc-shaped structure further avoids a damage to the inner wall of the sheath.

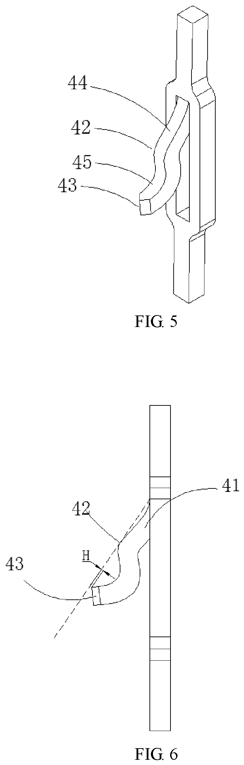

Referring to Fig. 5 and Fig. 6, in another embodiment, the overall barb structure 4 is "S"-shaped. The first section 44 is of a curved structure, and the second section 45 is also a curved structure. The free end 43 extends out towards the outside of the stent structure 1. The free end 43 is of a sharp end structure. An angle formed between a straight line formed by the connection end 41 and the first position 42 and a central axis of the stent structure 1 is greater than an angle formed between a straight line formed by the connection end 41 and the free end 43 and the central axis of the stent structure 1.

In this embodiment, the stent structure 1 is made of a nickel-titanium material. The barb structure 4 and the stent structure 1 are connected together after being respectively formed. For example, they can be welded together, or the barb structure 4 and the stent structure 1 are integrally formed by cutting a tubular product. The curved portion of the barb structure 4 can be clamped by tweezers and bent by hot blowing, the arc-shaped structure on the barb structure 4 can be made by grinding.

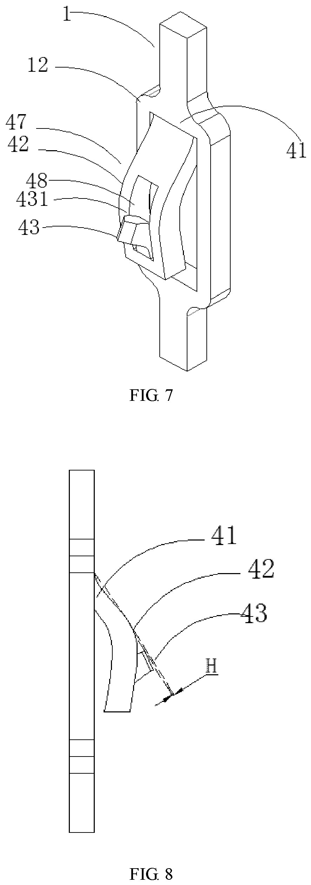

In another embodiment, referring to Fig. 7 to Fig. 8, the barb structure 4 includes an elastic element 47. The elastic element 47 can be accommodated in the mesh structure. For example, the elastic element 47 can be arranged in the first mesh unit 1 or the second mesh unit 12, or in any mesh structure on the stent structure 1.

In this embodiment, one end of the elastic element 47 is connected to the second mesh unit 12 to form a connection end 41. The elastic element 47 is configured to outwards protrude from the stent structure 1 to form a first position 42 and then bend from the first position 42 to the stent structure 1. A window structure 48 is arranged on the elastic element 47. A thorn-like structure 431 is arranged in the window structure 48. The thorn-like structure 431 has a free end 43. The free end 43 extends from the window structure 48 to a direction away from the stent structure 1. Similar to the above embodiment, an angle formed between a straight line formed by the connection end 41 and the first position 42 and a central axis of the stent structure 1 is greater than an angle formed between a straight line formed by the connection end 41 and the free end 43 and the central axis of the stent structure 1.

In this embodiment, the elastic element 47 and the free end 43 of the thorn-like structure 431 bend in opposite directions. Only the elastic element 47 compresses and expands during the releasing and loading of the stent structure 1. The bending angle of the thorn-like structure 431 remains unchanged, which can keep a good piercing direction. In this embodiment, the stent structure can be a radially compressible or expandable structure or a structure without radial compression or expansion.

In addition, the connection end 41 is connected to the distal end (close to the blood outflow end) of the second mesh unit 12. The thorn-like structure 431 is connected to the proximal end (close to the blood inflow end) of the window structure 48. Or the connection end 41 is connected to the proximal end (close to the blood inflow end) of the second mesh unit 12, and the thorn-like structure 431 is connected to the distal end (close to the blood outflow end) of the window structure 48. In this embodiment, the window structure 48 is rectangular. In other embodiments, the window structure 48 can be of any shape, such as triangle, circle, ellipse or polygon, as long as the free end 43 can extend out from the window structure 48. In this embodiment, other features are the same as those in other embodiments and will not be repeated here.

The above descriptions are only various implementation modes of the embodiments, but the scope of the embodiments is not limited to these implementations. Changes or substitutions that can be considered by any person of ordinary skill in the art without departing from the scope of the embodiments shall all fall within the scope of the embodiments.

## Claims

1. An implant, comprising: a tubular stent structure and a skirt, wherein the stent structure is provided with at least one barb structure; the stent structure is radially compressible or expandable; the stent structure comprises mesh structures that are circumferentially distributed; the skirt is arranged on a surface of the stent structure; the mesh structure comprise a plurality of first mesh units and a plurality of second mesh units; an area of one of the first mesh units is larger than an area of one of the second mesh units; a size of one of the second mesh units is substantially the same as a size of one barb structure; and one barb structure is accommodated in the one of second mesh units.

2. The implant according to claim 1, wherein a junction is provided between two adjacent first mesh units; and each second mesh unit is arranged at the junction.

3. The implant according to claim 2, wherein the first mesh units are hexagonal structures; and one edge of one hexagonal structure overlaps one edge of another hexagonal structure to form the junction.

4. The implant according to claim 1, wherein each first mesh unit is provided with at least one arc-shaped bulge structure along an axial direction of the stent structure; and the arc-shaped bulge structure protrudes in a direction away from the first mesh unit.

5. The implant according to claim 2, wherein each barb structure comprises a connection end, a first position, and a free end; the connection end is connected to the junction; the free end extends out of the second mesh unit; the first position is located between the connection end and the free end; and an angle formed between a straight line formed by the connection end and the first position and a central axis of the stent structure is greater than an angle formed between a straight line formed by the connection end and the free end and the central axis of the stent structure.

6. The implant according to claim 5, wherein the barb structure comprises a first section and a second section connected to each other; the first section is between the connection end and the first position; the second section is between the first position and the free end; and the free end is a sharp end structure.

7. The implant according to claim 6, wherein the first section is a straight bar-shaped structure, and a first surface of the second section facing an outer side of the stent structure is provided with an arc-shaped structure.

8. The implant according to claim 6, wherein the first section is a straight bar-shaped structure or a curved structure; and the second section is a curved structure.

9. The implant according to claim 1, wherein the barb structures and the stent structure are connected together after being formed respectively; or the barb structures and the stent structure are integrally formed by cutting a tubular product.

10. The implant according to claim 1, wherein the implant is a heart valve; the heart valve further comprises a valve structure; the valve structure comprises a plurality of valve leaflets fitting one another; each valve leaflet comprises a flange; and the flange passes through the second mesh unit in a direction from the inside of the stent structure to the outside.

11. An implant, comprising: a tubular stent structure and at least one barb structure arranged on the stent structure, wherein the stent structure comprises a plurality of mesh structures circumferentially distributed; one barb structure is accommodated in one of the mesh structures; each barb structure comprises an elastic element;
one end of each elastic element is connected to each mesh structure to form a connection end;
the elastic element is configured to protrude outwardly from the stent structure to form a first position and then bend from the first position to the stent structure;
a window structure is arranged on the elastic element; a thorn-like structure connected to the window structure is arranged in the window structure; the thorn-like structure has a free end extending from the window structure to a direction away from the stent structure; and an angle formed between a straight line formed by the connection end and the first position and a central axis of the stent structure is greater than an angle formed between a straight line formed by the connection end, and the free end and the central axis of the stent structure.

12. The implant according to claim 11, wherein the connection ends are connected to distal ends of the mesh structures, and the thorn-like structures are connected to proximal ends of the window structures; or the connection ends are connected to proximal ends of the mesh structures, and the thorn-like structures are connected to distal ends of the window structures.

13. The implant according to claim 11, wherein the mesh structures comprise a plurality of first mesh units and a plurality of second mesh units; an area of one of the first mesh units is larger than an area of one of the second mesh units; a size of one of second mesh units is substantially the same as a size of one barb structure; and one barb structure is accommodated in the one of second mesh units.

14. The implant according to claim 13, wherein a junction is provided between two adjacent first mesh units; and each second mesh unit is arranged at the junction.

15. The implant according to claim 14, wherein the first mesh units are hexagonal structures; and one edge of one hexagonal structure overlaps one edge of the other hexagonal structure to form the junction.

16. The implant according to claim 13, wherein the stent structure is radially compressible or expandable and when the stent structure radially compresses or expands, the first mesh units circumferentially compress or expand along the stent structure; each first mesh unit is provided with at least one arc-shaped bulge structure along an axial direction of the stent structure; and the arc-shaped bulge structure protrudes in a direction away from the first mesh unit.

17. The implant according to claim 11, wherein the free end is a sharp end structure.

18. The implant according to claim 11, wherein at least one barb structure and the stent structure are connected into a whole after being respectively formed.

19. The implant according to claim 11, wherein at least one barb structure and the stent structure are integrally formed by cutting a tubular product.

20. The implant according to claim 13, wherein the implant is a heart valve; the heart valve further comprises a valve structure; the valve structure comprises a plurality of valve leaflets fitting one another; each valve leaflet comprises a flange; and the flange passes through the second mesh unit in a direction from the inside of the stent structure to the outside.
